# EUROPÄISCHE PATENTSCHRIFT

(11) **EP 2 566 844 B1**
(45) Veröffentlichungstag und Bekanntmachung des Hinweises auf die Patenterteilung: **15.03.2017**
(21) Anmeldenummer: 11718339.2
(22) Anmeldetag: 29.04.2011
(51) Int. Cl.: C07C 263/10

(54) **VERFAHREN ZUR HERSTELLUNG VON ISOCYANATEN IN DER GASPHASE**
METHOD FOR PRODUCING ISOCYANATES IN THE GAS PHASE
PROCÉDÉ DE PRÉPARATION D'ISOCYANATES EN PHASE GAZEUSE

(30) Priorität: 05.05.2010 DE 102010019342
(43) Veröffentlichungstag der Anmeldung: 13.03.2013
(73) Patentinhaber: Covestro Deutschland AG, 51373 Leverkusen (DE)
(72) Erfinder: BRUNS, Rainer, 51373 Leverkusen (DE); LORENZ, Wolfgang, 41540 Dormagen (DE); RAUSCH, Andreas, Karl, 41564 Kaarst (DE); WERSHOFEN, Stefan, 41065 Mönchengladbach (DE); LODDENKEMPER, Tim, 41542 Dormagen (DE)
(74) Vertreter: Levpat
(86) Internationale Anmeldenummer: PCT/EP2011/056848
(87) Internationale Veröffentlichungsnummer: WO 2011/138245

(56) Entgegenhaltungen:
- EP-A1- 2 060 560
- WO-A1-2009/027232
- WO-A1-2009/077795
- WO-A1-2010/121997
- US-A1- 2009 221 846

## Beschreibung

Die Erfindung betrifft ein Verfahren zur Herstellung von Isocyanaten durch Umsetzung der korrespondierenden Amine mit Phosgen in der Gasphase, worin bei der Überführung des Amins in die Gasphase ein Verdünnungsmittel, enthaltend zwischen 90,0000 Massen-% und 99,9999 Massen-% an im Phosgenierprozess inerten Stoffen und zwischen 0,0001 Massen-% und 10,0000 Massen-% Sauerstoff (O₂), zugegen ist und das molare Verhältnis von Amin zu Sauerstoff (O₂) ≥ 1.000 : 1 ist.

Isocyanate und insbesondere Diisocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Die Umsetzung der Amine mit dem Phosgen kann in der Flüssigphase oder durch Phosgenierung in der Gasphase erfolgen.

Die vorliegende Erfindung betrifft ausschließlich die Phosgenierung in der Gasphase.

Diese Verfahrensführung zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Reaktionsbedingungen gasförmig sind. Vorteile der Gasphasenphosgenierung sind unter anderem ein verringerter Phosgen-Hold-Up, die Vermeidung schwer phosgenierbarer Zwischenprodukte sowie erhöhte Reaktionsausbeuten.

Es sind aus dem Stand der Technik verschiedene Verfahren zur Herstellung von Diisocyanaten durch Umsetzung von Diaminen mit Phosgen in der Gasphase bekannt.

EP 0 289 840 B1 offenbart erstmals ein Verfahren zur Herstellung aliphatischer Diisocyanate durch Phosgenierung der entsprechenden Diamine in der Gasphase, in welchem die dampfförmigen Diamine, gegebenenfalls verdünnt mit einem Inertgas oder mit den Dämpfen eines inerten Lösungsmittels, und Phosgen getrennt voneinander auf Temperaturen von 200 °C bis 600 °C erhitzt und kontinuierlich in einem auf 200 °C bis 600 °C erhitzten Reaktionsraum miteinander zur Reaktion gebracht werden. Bevorzugtes Inertgas ist Stickstoff. Als geeignete inerte Lösungsmittel, deren Dämpfe ebenfalls zur Verdünnung des Diamins verwendet werden können, werden Monochlorbenzol, ortho-Dichlorbenzol, Xylol, Chlornaphtalin, Decahydronaphtalin oder deren Gemische genannt. Gemäß dieser Schrift ist die Menge des gegebenenfalls als Verdünnungmittel mitverwendeten Inertgases oder Lösungsmitteldampfes nicht kritisch. Falls eine Verdünnung des Diamins stattfindet, kann dies beispielsweise unter Einhaltung eines Volumenverhältnisses von Diamindampf zu Inertgas bzw. Lösungsmitteldampf von 1 : 0,5 bis 1 : 2 erfolgen. Über die Forderung hinaus, dass das Verdünnungsmittel sich inert verhalten soll, werden keine weiteren Angaben zu dessen Reinheit gemacht.

EP 0 593 334 B1 beschreibt erstmals ein Verfahren zur Herstellung aromatischer Diisocyanate in der Gasphase. Hier können die bei der Reaktion in Kontakt gebrachten Reaktanten, d. h. mindestens ein Diamin und Phosgen, allein oder in Gegenwart eines verdünnenden Gasstroms eingesetzt werden. Unter einem verdünnenden Gasstrom ("Gasverdünnungsvektor") wird dabei jedes Gas verstanden, das verdünnend ist und gegenüber den Reaktionsteilnehmern und Reaktionsprodukten inert ist. Außer den inerten Gasen, wie insbesondere Stickstoff, kann auch der Dampf eines Lösungsmittels, insbesondere Benzol, Xylol, Monochlorbenzol oder ortho-Dichlorbenzol verwendet werden. Über die Forderung hinaus, dass sich der verdünnende Gasstrom inert verhalten soll, werden keine weiteren Angaben zu dessen Reinheit gemacht.

EP 2 060 560 A1 offenbart ein Verfahren zur Herstellung von Isocyanaten durch Umsetzung der korrespondierenden Amine mit Phosgen in der Gasphase, wobei ein inertes Verdünnungsmittel anwesend sein kann (Absätze [0019] und [0020]). Die Reinheit des Verdünnungsmittels bzw. Anwesenheit von Sauerstoff im Verdünnungsmittel wird nicht diskutiert.

WO 2009/027232 A1 offenbart ein Verfahren zur Herstellung von Isocyanaten durch Umsetzung der korrespondierenden Amine mit Phosgen in der Gasphase in Gegenwart eines inerten Verdünnungsmittels (Seite 11, Zeile 1 bis Zeile 19). Der Inertstrom wird in einer Mischeinrichtung zwischen einen Phosgenstrom und einen Aminstrom eindosiert (vgl. die Zeichnungen der Anmeldung).

US 2009/221846 A1 offenbart ein Verfahren zur Herstellung von Isocyanaten durch Umsetzung der korrespondierenden Amine mit Phosgen in der Gasphase, ggf. in Gegenwart eines inerten Verdünnungsmittels (Absätze [0033] und Absatz [0037]. Amin- und Phosgenstrom werden dem Reaktor über Ringspalte zugeführt. Die Reinheit des Verdünnungsmittels bzw. Anwesenheit von Sauerstoff im Verdünnungsmittel wird nicht diskutiert.

Der Einsatz von Verdünnungsmitteln (Inertgase oder die Dämpfe inerter Lösungsmittel) zur Verdünnung der Edukte Amin und / oder Phosgen ist somit Stand der Technik. Insbesondere die Verdünnung des Amins ist allgemeine Praxis. Der Grund hierfür besteht darin, dass geeignete inerte Stoffe, häufig Stickstoff, als Schleppmittel wirken und die Verdampfung des Amins erleichtern, wodurch Zersetzungsreaktionen (beispielsweise unter Ammoniakabspaltung) verringert werden. So bewirkt beispielsweise der Zusatz bereits geringer Mengen an Stickstoff zu Toluylendiamin (in der Regel ein Gemisch verschiedener Isomere, im Folgenden summarisch mit TDA abgekürzt) eine signifikante Verringerung der Verdampfungstemperatur. Beispielsweise führt die Zugabe von 4 Massen-% Stickstoff eine Verringerung der Verdampfungstemperatur des TDA um ca. 8 K. Somit reichen bereits geringe Mengen eines Verdünnungsmittels aus, um die Verdampfungstemperatur abzusenken. Der zur Siedepunktserniedrigung zugegebene Stoff soll sich gegenüber dem Amin und den späteren Reaktionsprodukten und/oder -zwischenprodukten inert verhalten, weil eine Reaktion des Amins und/oder der Reaktionsprodukte und/oder der Reaktionszwischenprodukte mit dem Hilfsstoff einerseits die Ausbeute bezogen auf Amin schmälern würde und andererseits zur Bildung von Ablagerungen führen und / oder die destillative Reinigung des Isocyanates erschweren könnte. Auch ist bekannt, dass oxidierend wirkende Stoffe die Eigenschaften eines Isocyanats so verändern können, dass im Isocyanat selbst oder im resultierenden Polyurethanschaum Verfärbungen auftreten (siehe z. B. WO 2005/010066, Seite 1, Zeile 24 bis 26).

Gemäß dem Stand der Technik ist Stickstoff eines der bevorzugt eingesetzten Verdünnungsmittel zur Absenkung der für die Verdampfung des Amins erforderlichen Temperatur. Technischer Stickstoff ist in unterschiedlichen Reinheiten erhältlich und durch unterschiedliche Herstellverfahren zugänglich. In der Mehrzahl der Verfahren zur Gewinnung von Stickstoff wird Luft als Einsatzstoff verwendet. Häufige Verfahren der Luftzerlegung sind Tiefkältedestillation, Membranverfahren und Druckwechseladsorption. Die Tiefkältedestillation ist die effektivste Technologie zur Produktion großer Mengen von Stickstoff. Druckwechseladsorption und Membranverfahren gelten als die ökonomischeren Verfahren für die Produktion von weniger reinem Stickstoff (bis zu 99,8 % Reinheit mit minimal 0,2 % verbleibendem Sauerstoffgehalt) bei mittleren Volumenströmen (25 - 500 Nm³/h). Um höhere Reinheiten zu erzielen, muss ein zusätzliches Deoxo-System zur Sauerstoffentfernung durch Reaktion mit Wasserstoff mit anschließender Gastrocknung eingesetzt werden. Derartige Deoxo-Systeme können jedoch dazu führen, dass Wasserstoff im Stickstoff verbleibt, und sie sind mit siginifikanten Kosten für die Wasserstoffversorgung und die Deoxo-Anlage selbst verbunden (Kirk-Othmer Encyclopedia of Chemical Technology, 2001 by John Wiley & Sons, Inc., Kapitel "Nitrogen"). Somit hat die benötigte Stickstoffmenge und vor allem die erforderliche Stickstoffreinheit einen wesentlichen Einfluss auf die Auswahl des Verfahrens zur Herstellung des Stickstoffs (siehe ebendort, Abbildung 1) und damit auf die Kosten, die für die Stickstoffversorgung zu berücksichtigen sind, wenn Stickstoff als Verdünnungsmittel in einem Verfahren zur Herstellung von Isocyanaten in der Gasphase eingesetzt werden soll. Sobald Reinheiten von weniger als 99,9 Vol.-% zulässig sind, stehen mehrere Verfahren zur Erzeugung des Stickstoffs zur Auswahl. Ist sogar eine Reinheit von weniger als 93 Vol.-% zulässig, so bietet sich der Vorteil, dass eine Tiefkältedestillation in einem Einsäulenapparat eingesetzt werden kann, die Stickstoff mit einem Sauerstoffgehalt von 7 Vol.-% erzeugt (Linde AG: Kryogene Luftzerlegung, Entstehung und technische Entwicklung).

Ebenfalls nach dem Stand der Technik als Verdünnungsmittel einsetzbar sind Edelgase, wobei insbesondere Argon zu nennen ist, das das preiswerteste Edelgas darstellt. Argon wird ebenso wie Stickstoff durch Luftzerlegung, insbesondere durch Tiefkältedestillation gewonnen. Da Argon nur 3 K tiefer siedet als Sauerstoff, gelingt die Herstellung von sauerstoffarmen Argon wiederum nur durch die Anwendung nachgeschalteter Schritte zur Sauerstoffentfernung, wie selektive Adsorption, Reaktion mit heißen Metall oder durch katalysierte Reaktion mit Wasserstoff zu Wasser und nachfolgender Trocknung. Durch diese Schritte kann die Argonreinheit zwar von 98 % nach der Tiefkältedestillation auf bis zu 99,999 % gesteigert werden, jedoch sind damit hohe Kosten für die zusätzlichen Anlagen und deren Betrieb und Instandhaltung erforderlich (Kirk-Othmer Encyclopedia of Chemical Technology, 2001 by John Wiley & Sons, Inc., Chapter "Nobel Gases").

"Inerte" organische Lösungsmittel enthalten in technisch üblichen Reinheitsgraden ebenfalls gewisse Mengen an gelöstem Sauerstoff. Ohne besondere Maßnahmen ist daher auch in den Dämpfen organischer Lösungsmittel, welche nach dem Stand der Technik als die Verdampfung des Amins erleichternde Verdünnungsmittel eingesetzt werden können, Sauerstoff vorhanden.

Dem Fachmann ist bekannt, dass sich Sauerstoff nicht inert gegenüber Aminen verhält, sondern es zu einer schnellen und irreversiblen Reaktion kommen kann, die zur Verfärbung des Amins führt. So beschreibt EP 0 954 549 B1 in Abschnitt [0002], dass Aminogruppen enthaltende Verbindungen generell dazu neigen, sich bei Kontakt mit Sauerstoff zu verfärben. So ist zum Beispiel für ortho-Toluoldiamin, wie 2,3-Diaminotoluol oder 3,4-Diaminotoluol, angegeben, dass sich dieses bereits unmittelbar nach der Einwirkung von Luft dunkel verfärbt, währende andere Amine, z. B. Anilin, zwar stabiler sind, jedoch im Laufe der Zeit ebenfalls dunkel werden. Aliphatische Verbindungen mit Aminogruppen verfärben sich auch im Laufe der Zeit bei Raumtemperatur; im Allgemeinen aber verfärben diese sich laut EP 0 954 549 B1 mit einer sehr viel geringeren Geschwindigkeit als aromatische Aminogruppen enthaltende Verbindungen.

WO 2009/077795 A1 offenbart ein Verfahren zur Herstellung von Isocyanaten, bei dem die Isocyanate einer thermischen Behandlung zur Entfernung halogenierter Verunreinigungen unterzogen werden. In diesem Zusammenhang wird gelehrt, dass sich Sauerstoff im Amin negativ auf die Farbe des Isocyanats auswirkt, mithin das Amin sauerstofffrei gehalten werden sollte (Seite 2, Zeile 16 bis Zeile 21).

Der Fachmann entnimmt somit dem Stand der Technik die allgemeine Lehre, dass im Falle des Einsatzes eines Verdünnungsmittels zur Erleichterung der Verdampfung des Amins dieses Verdünnungsmittel eine sehr hohe Reinheit, insbesondere einen sehr niedrigen Gehalt an Sauerstoff aufweisen muss, da es sonst zu Nebenreaktionen des Amins mit dem Sauerstoff kommt.

Diese Beschränkungen sind nachteilig, da dadurch einerseits die Kosten für Bezug und Qualitätsüberwachung des Verdünnungsmittels, z. B. Stickstoff oder Argon steigen und andererseits die Flexibilität des Verfahrens zur Herstellung von Isocyanaten in der Gasphase leidet, da das einzusetzende Verdünnungsmittel in hoher Reinheit zur Verfügung stehen muss, was je nach Standort nur mit hohem logistischem Aufwand realisierbar ist.

Aufgabe der vorliegenden Erfindung war es daher, ein Verfahren zur Herstellung von Isocyanaten durch Umsetzung der korrespondierenden Amine mit Phosgen in der Gasphase bereitzustellen, in welchem ein im Phosgenierprozess inerter Stoff eingesetzt werden kann, um die Überführung des Amins in die Gasphase zu erleichtern, ohne dass die Reinheitsanforderungen an den im Phosgenierprozess inerten Stoff die Wirtschaftlichkeit des Verfahrens negativ beeinflussen.

Überraschend wurde gefunden, dass die Aufgabe gelöst werden kann durch ein Verfahren zur Herstellung von Isocyanaten durch Umsetzung der korrespondierenden Amine mit Phosgen in der Gasphase, worin
(i) das Amin in einem Verdampfungsraum in Gegenwart von > 0,10 Massen-% bis 25 Massen-%, bevorzugt von > 0,10 Massen-% bis 10 Massen-%, besonders bevorzugt von > 0,20 Massen-% bis 5,0 Massen-% eines Verdünnungsmittels enthaltend mindestens einen im Phosgenierprozess inerten Stoff, bezogen auf die Gesamtmasse aus Verdünnungsmittel und Amin, in die Gasphase überführt wird;
(ii) der aus (i) erhaltene, Amin und Verdünnungsmittel enthaltende gasförmige Strom mit einem gasförmigen Phosgenstrom in einem Reaktionsraum zum entsprechenden Isocyanat umgesetzt wird;
wobei
das in Schritt (i) eingesetzte Verdünnungsmittel zwischen 90,0000 Massen-% und 99,9999 Massen-%, bevorzugt zwischen 99,0000 Massen-% und 99,9950 Massen-%, besonders bevorzugt zwischen 99,5000 Massen-% und 99,9900 Massen-% an im Phosgenierprozess inerten Stoffen und zwischen 0,0001 Massen-% und 10,0000 Massen-%, bevorzugt zwischen 0,0050 Massen-% und 1,0000 Massen-%, besonders bevorzugt zwischen 0,0100 Massen-% und 0,5000 Massen-% Sauerstoff, jeweils bezogen auf die Masse des Verdünnungsmittels, enthält, und
das molare Verhältnis von Amin zu Sauerstoff (O₂) in Schritt (i) im Verdampfungsraum ≥ 1.000 : 1 ist und bevorzugt im Bereich zwischen 1.000 : 1 und 1.000.000 : 1, besonders bevorzugt zwischen 10.000 : 1 und 50.000 : 1, liegt.

Unter "Umsetzung in der Gasphase" ist zu verstehen, dass die Amine im gasförmigen Zustand zu den Isocyanaten reagieren und im Verlauf der Reaktion sämtliche vorhandenen Komponenten (Edukte, Produkte, Zwischenprodukte, ggf. Nebenprodukte, ggf. Inertstoffe) während des Durchgangs durch den Reaktionsraum zu mindestens 95,0 Massen-%, bevorzugt zu mindestens 98,0 Massen-%, besonders bevorzugt zu mindestens 99,0 Massen-% und ganz besonders bevorzugt zu mindestens 99,9 Massen-%, jeweils bezogen auf die Gesamtmasse aller vorhandenen Komponenten, in der Gasphase verbleiben.

Unter "Verdampfungsraum" wird dabei der Raum verstanden, in welchem das primäre Amin in die Gasphase überführt wird. Der Verdampfungsraum befindet sich in einer technischen Vorrichtung zur Überführung von Flüssigkeiten in die Gasphase, dem *Verdampfer*. Im einfachsten Fall ist der Verdampfungsraum identisch mit dem Innenvolumen des Verdampfers.

Bei einem "im Phosgenierprozess inerten Stoff" (im Folgenden vereinfacht als "inerter Stoff" bezeichnet) im Sinne der Erfindung handelt es sich um einen Stoff, der bei der Reaktionstemperatur gasförmig im Reaktionsraum vorliegt und als solcher (d. h. bei - in der Praxis nicht erreichbarer - 100%iger Reinheit) bevorzugt überhaupt nicht oder nur unwesentlich (d. h. ohne messbare Beeinträchtigung des Verfahrens) mit den im Reaktionsverlauf auftretenden Verbindungen (Amin, Phosgen, Zwischenprodukte, Nebenprodukte, Produkte) reagiert. Der inerte Stoff bildet zusammen mit in ihm enthaltenen Verunreinigungen (in der Regel ausschließlich oder zumindest überwiegend Sauerstoff) das *Verdünnungsmittel*. Es können auch Gemische mehrerer im Phosgenierprozess inerter Stoffe eingesetzt werden.

Unter "Reaktionsraum" wird dabei der Raum verstanden, in welchem die Gasphasenreaktion von primärem Amin (bzw. Zwischenprodukten) mit Phosgen zum gewünschten Isocyanat stattfindet. Dieser Raum beginnt dort, wo die Edukte erstmalig vermischt werden und endet dort, wo die Vorraussetzungen für eine Gasphasenreaktion von primärem Amin (bzw. Zwischenprodukten) mit Phosgen zum gewünschten Isocyanat nicht mehr gegeben sind (beispielsweise durch Abbruch der Reaktion infolge Temperaturerniedrigung oder "Quenche" (siehe weiter unten) etc.).

Die erfindungsgemäße Lösung der Aufgabe ist insbesondere überraschend, weil bekannt ist, dass Sauerstoff mit Aminen reagiert und somit keinesfalls eine inerte Verbindung darstellt bzw. sich nicht inert verhält. Aufgrund dieses Befundes kann der Stand der Technik der Gasphasenphosgenierung verbessert werden, da als Verdünnungsmittel bei der Überführung des Amins in die Gasphase nun ein Stoff geringerer als bisher üblicher Reinheit (d. h. mit höheren Sauerstoffgehalten) eingesetzt werden kann, wodurch die Wirtschaftlichkeit des Verfahrens verbessert wird. Zudem ist die erfindungsgemäße Lösung der Aufgabe überraschend, weil bekannt ist, dass Sauerstoff ebenfalls mit dem Reaktionsprodukt Isocyanat reagiert und sich somit gegenüber dem Reaktionsprodukt nicht inert verhält. Das erfindungsgemäße Verdünnungsmittel ist somit ein Gemisch aus dem eigentlichen inerten Stoff und Sauerstoff in geringen Mengen.

Es wurde nämlich gefunden, dass Sauerstoff in kleinen Mengen, nämlich in Mengen von weniger als ein 1000stel der Stoffmenge des Amins im Verdampfungsraum, zwar in Übereinstimmung mit dem Stand der Technik sehr schnell mit dem Amin reagiert, diese Reaktion aber das Verfahren zur Herstellung von Isocyanaten in der Gasphase nur unmaßgeblich beeinflusst.

Enthält der Strom an gasförmigem Amin Sauerstoff in einer Stoffmenge [mol], die kleiner ist als ein 1000stel der Stoffmenge des verdampften Amins [mol], so führt die Reaktion des Sauerstoffs mit dem Amin zur Bildung von monomeren und binären Oxidationsprodukten, deren Siedepunkte nicht wesentlich höher liegen (d. h. nur um wenige K, im Allgemeinen nicht mehr als 20 K, bevorzugt nicht mehr als 10 K, besonders bevorzugt nicht mehr als 5 K) als der des ursprünglichen Amins. *Somit verbleiben die monomeren und binären Oxidationsprodukte in der Gasphase* und treten zusammen mit dem Aminstrom in den Reaktionsraum zur Gasphasenphosgenierung ein. Dort reagieren die monomeren und binären Oxidationsprodukte mit Phosgen zu teilweise chlorhaltigen Folgeprodukten, die höher sieden als das entsprechende Isocyanat, sodass diese Folgeprodukte der monomeren und binären Oxidationsprodukte im so genannten "hoch siedenden Rückstand" des Isocyanatverfahrens verbleiben. Die Anwesenheit von kleinen Mengen an Sauerstoff führt somit zu einer leichten Verringerung an Ausbeute in Bezug auf das Amin, zu einer leicht erhöhten Bildung an Isocyanat-Rückstand, aber *nicht zu einer Beeinträchtigung der Verdampfung des Amins oder der Zuführung des gasförmigem Amins in den Reaktionsraum*. Diese geringfügigen Ausbeuteverluste werden durch den günstigeren Bezug und ggf. bei Recyclisierung des inerten Stoffes durch den geringeren Verbrauch in wirtschaftlicher Hinsicht mehr als ausgeglichen.

Enthält der Strom an verdampften Amin Sauerstoff in einer Stoffmenge [mol], die größer oder gleich 1000stel der Stoffmenge des verdampften Amins [mol] ist, so führt die Reaktion des Sauerstoffs mit dem Amin neben den monomeren und binären Oxidationsprodukten auch zu hoch siedenden oligomeren Oxidationsprodukten. Der Anteil an oligomeren Oxidationsprodukten bis hin zu teerartigen Produkten steigt, je weiter der Sauerstoffanteil in dem gasförmigen Amin ansteigt. Die oligomeren Oxidationsprodukte weisen einen Siedepunkt auf, der deutlich oberhalb des Siedepunktes des ursprünglichen Diamins liegt. Somit kann es zur Kondensation und Verstopfung der Leitungen, welche gasförmiges Amine enthalten, kommen. Verstopfungen oder druckverlustverursachende Querschnittsverengungen haben massive Auswirkungen auf die Standzeit des Gasphasenreaktors und auf die Verfügbarkeit des Verfahrens zur Herstellung von Isocyanaten in der Gasphase und sind äußerst kritisch zu bewerten. Hinzu kommt, dass mit zunehmender Oxidation des Amins auch Wasser als Produkt der Oxidation gebildet werden kann. Die Anwesenheit von Wasser ist in der Phosgenierreaktion ebenfalls unerwünscht, da es hierdurch zur Zersetzung des Phosgens unter Chlorwasserstoffbildung kommt und somit die innerhalb des Reaktionsraumes angestrebte Stöchiometrie negativ beeinflusst wird. Außerdem sind Phosgenverluste die Folge. Weiterhin ist Wasser in Phosgenierreaktionen unerwünscht, da sich daraus die Gefahr von Korrosion ergibt, sofern nicht besonders hochwertige (und daher besonders teure) Werkstoffe eingesetzt werden.

In einer bevorzugten Ausführung der vorliegenden Erfindung wird das Amin zum Zwecke der Gasphasenphosgenierung in mindestens einem Verdampfungsraum in Gegenwart eines Verdünnungsmittels enthaltend einen inerten Stoff in die Gasphase überführt und dabei auf Temperaturen von 200 °C bis 600 °C, bevorzugt 200 °C bis 500 °C, besonders bevorzugt 250 °C bis 450 °C erhitzt und dem Reaktionsraum zugeführt. Erfindungsgemäß wird dabei ein Verdünnungsmittel verwendet, das zwischen 90,0000 Massen-% und 99,9999 Massen-%, bevorzugt zwischen 99,0000 Massen-% und 99,9950 Massen-%, besonders bevorzugt zwischen 99,5000 Massen-% und 99,9900 Massen-% an im Phosgenierprozess inerten Stoffen und zwischen 0,0001 Massen-% und 10,0000 Massen-%, bevorzugt zwischen 0,0050 Massen-% und 1,0000 Massen-%, besonders bevorzugt zwischen 0,0100 Massen-% und 0,5000 Massen-% Sauerstoff, jeweils bezogen auf die Masse des Verdünnungsmittels, enthält. Wird ein Gemisch aus mehreren inerten Stoffen eingesetzt (z. B. Stickstoff und ein Edelgas), so beziehen sich die oben genannten Massenanteile "an inertem Stoff" auf die Summe an allen inerten Stoffen. Das Verdünnungsmittel wird in Gehalten von 0,10 Massen-% bis 25 Massen-%, bevorzugt von 0,10 Massen-% bis 10 Massen-%, besonders bevorzugt von 0,20 Massen-% bis 5,0 Massen-%, bezogen auf die Gesamtmasse an Amin und Verdünnungsmittel, eingesetzt, wobei ein molares Verhältnis von Amin zu Sauerstoff (O₂) von ≥ 1.000 : 1, bevorzugt im Bereich zwischen 1.000 : 1 und 1.000.000 : 1, besonders bevorzugt zwischen 10.000 : 1 und 50.000 : 1, eingehalten wird. Dabei spiegeln die Untergrenzen für das molare Verhältnis von Amin zu Sauerstoff jeweils technische Effekte wider (ein zu kleines Amin : Sauerstoff-Verhältnis führt zu den bereits weiter oben erwähnten negativen Auswirkungen), während die Obergrenzen für das molare Verhältnis von Amin zu Sauerstoff jeweils wirtschaftlichen Effekten geschuldet sind (ein zu großes Amin : Sauerstoff-Verhältnis erhöht den Preis für das Verdünnungsmittel unverhältnismäßig, wenn man ein sehr großes Amin : Sauerstoff-Verhältnis nicht dadurch erreichen will, dass man die Menge an Verdünnungsmittel stark verringert, was aber nicht unbegrenzt möglich ist, weil unterhalb einer bestimmten Grenzkonzentration, die im Allgemeinen bei etwa 0,10 Massen-% des Verdünnungsmittels, bezogen auf die Gesamtmasse von Verdünnungsmittel und zu verdampfenden Amin liegt, keine erleichterte Verdampfung mehr erreicht werden kann).

Als inerte Stoffe kommen dabei beispielsweise einerseits solche Stoffe in Frage, die bereits bei Raumtemperatur gasförmig vorliegen, also beispielsweise Stickstoff, Edelgase wie Helium oder Argon, andere Gase wie Kohlenstoffdioxid. Andererseits kommen - weniger bevorzugt - als inerte Stoffe auch solche in Frage, die erst bei Temperaturen oberhalb Raumtemperatur gasförmig vorliegen, also beispielsweise Aromaten wie Chlorbenzol, Chlortoluol (o-, m-, p-Isomere), Dichlorbenzol (o-, m-, p-Isomere), Toluol, Xylol (o-, m-, p-Isomere), Chlornaphthalin (alle Isomere) oder Decahydronaphthalin. In der Gruppe der bereits bei Raumtemperatur gasförmig vorliegenden Stoffe ist Stickstoff besonders bevorzugt, weil dieser die Kriterien hinsichtlich chemischer Inertheit äußerst gut erfüllt und dabei wesentlich preisgünstiger ist als Edelgase. In der Gruppe der erst oberhalb Raumtemperatur gasförmig vorliegenden Stoffe sind Stoffe bevorzugt, die auch als Lösemittel im Prozess verwendet werden. Insbesondere bevorzugt sind Chlorbenzol und Dichlorbenzol. Von allen denkbaren inerten Stoffen ist Stickstoff am stärksten bevorzugt.

In einer besonders bevorzugten Ausführung der vorliegenden Erfindung wird ein Verdünnungsmittel enthaltend einen bereits bei Raumtemperatur gasförmigen inerten Stoff, bevorzugt N₂, He, Ar, besonders bevorzugt N₂ eingesetzt. Das Verdünnungsmittel enthält außer dem inerten Stoff mindestens noch Sauerstoff, und zwar in Konzentrationen zwischen 0,0001 Massen-% und ≤ 10,0000 Massen-%, bevorzugt zwischen 0,0050 Massen-% und 1,0000 Massen-%, besonders bevorzugt zwischen 0,0100 Massen-% und 0,5000 Massen-%, bezogen auf die Masse des Verdünnungsmittels. Aufgrund der zulässigen geringen Reinheit des Verdünnungsmittels von lediglich ≥ 90,0000 %, bevorzugt ≥ 99,0000 %, besonders bevorzugt ≥ 99,5000 %, können preiswertere Verfahren zur Erzeugung des Verdünnungsmittels angewandt werden, wodurch sich die Bezugskosten für das Verdünnungsmittel verringern und sich die Wirtschaftlichkeit des Verfahrens zur Herstellung von Isocyanaten erhöht. Verdünnungsmittel mit einer geringeren Reinheit enthalten neben Sauerstoff oft auch andere Stoffe, die die Aminverdampfung nicht notwendigerweise stören. So kann z. B. Stickstoff geringerer Reinheit noch Argon, Helium und Wasser enthalten.

Im erfindungsgemäßen Verfahren wird bevorzugt der Sauerstoffgehalt des einzusetzenden Verdünnungsmittels vor dessen Einfuhr in den Verdampfungsraum ermittelt. Hierunter fällt auch die Kenntnisnahme von Analysenzertifikaten der jeweiligen Lieferanten der Verdünnungsmittel, falls diese vom Isocyanathersteller ohne eigene Analyse zugekauft werden. Wenn als Verdünnungsmittel sauerstoffhaltige Gase wie Stickstoff oder Argon, bevorzugt Stickstoff, eingesetzt werden, so wird der Sauerstoffgehalt des Verdünnungsmittels bevorzugt durch eine dem Fachmann geläufige gaschromatographische Online-Analytik ermittelt.

Ergibt die Analyse des Sauerstoffgehalts des Verdünnungsmittels, dass die Einspeisung eines ursprünglich angestrebten Mengenstromes dieses Verdünnungsmittel in den Verdampfungsraum bei gegebenem Mengenstrom an Amin zu einem molaren Verhältnis von Amin zu Sauerstoff von kleiner als 1.000 : 1, bevorzugt kleiner als 10.000 : 1, führen würde, so ergeben sich folgende Möglichkeiten:
a) Verringerung des Mengenstroms an Verdünnungsmittel unter der Randbedingung, dass im Verdampfungsraum der Minimalgehalt von > 0,10 Massen-% Verdünnungsmittel, bezogen auf die Gesamtmasse von Amin und Verdünnungsmittel, nicht unterschritten wird. In dieser Ausführungsform wird demnach die Einhaltung des molaren Verhältnisses von Amin zu Sauerstoff (O₂) in Schritt (i) (Überführung des Amins in die Gasphase) im Verdampfungsraum von ≥ 1.000 : 1, bevorzugt im Bereich zwischen 1.000 : 1 und 1.000.000 : 1, besonders bevorzugt zwischen 10.000 : 1 und 50.000 : 1, durch Verringerung des absoluten Mengenstromes des dem Verdampfungsraum zugeführten Verdünnungsmittels erzielt (siehe hierzu auch Beispiel 3).
   *Oder:*
b) Vermischen der Charge des Verdünnungsmittels zu geringer Reinheit mit einer anderen, reineren Charge des Verdünnungsmittels in einem solchen Verhältnis, dass die Einspeisung des ursprünglich angestrebten Mengenstromes an Verdünnungsmittel in den Verdampfungsraum mit der so erhaltenen Mischcharge vorgenommen werden kann, ohne das minimale molare Verhältnis von Amin zu Sauerstoff von 1000 : 1 zu unterschreiten. In dieser Ausführungsform ist demnach das in Schritt (i) (Überführung des Amins in die Gasphase) eingesetzte Verdünnungsmittel eine Mischung aus verschiedenen Chargen des Verdünnungsmittels mit unterschiedlichen Sauerstoffgehalten. Die reinere Charge des Verdünnungsmittels kann zum Beispiel dadurch erhalten werden, dass das Verdünnungsmittel, das den Reaktionsraum bereits passiert hat, und dessen enthaltender Sauerstoff durch Reaktion mit Amin oder anderen in der Reaktionsmischung vorhandenen Stoffen eliminiert wurde, wiedergewonnen und zurückgeführt wird.

Als Verdampfer für die Aminverdampfung können grundsätzlich beliebige geeignete Verdampfer eingesetzt werden. Bevorzugt können Rohrbündelwärmetauscher, Plattenwärmetauscher oder Fallfilmverdampfer, gegebenenfalls mit Umpumpkreislauf, eingesetzt werden. Auch können Mikrowärmetauscher oder Mikroverdampfer wie in WO 2005/016512 A oder in DE 10 2005 036870 A1 beschrieben, eingesetzt werden. Bevorzugt werden Verdampfungssysteme eingesetzt, bei denen ein kleiner Arbeitsinhalt mit einer hohen Umwälzleistung über einen Fallfilmverdampfer geführt wird. Bevorzugt werden weiterhin solche Verdampfungssystem eingesetzt, bei denen ein kontinuierlicher Flüssigkeitsaustrag aus dem Verdampfer erfolgt.

Die erfindungsgemäße Überführung von Aminen in die Gasphase hat in erster Linie für die Gasphasenphosgenierung der Amine zu den korrespondierenden Isocyanaten Bedeutung. Grundsätzlich können die Überlegungen zur Reinheit des Verdünnungsmittels jedoch auch auf andere Anwendungen der Amin-Verdampfung übertragen werden. Im Falle des TDA umfassen andere Anwendungen der Amin-Verdampfung z. B. die Verdampfung zum Zwecke der Destillation, wie zum Beispiel der Trennung von ortho- und meta-TDA-Isomeren, oder der destillativen Abtrennung von TDA-Rückstand. Andere Anwendungen der TDA-Verdampfung können auch Anwendungen umfassen, bei denen TDA nur teilweise verdampft wird, z. B. bei dem Austreiben von gelösten Gasen, wie zum Beispiel Ammoniak, mit einem Inertgas. Teilweise Verdampfung ist auch bei Lagerung und Transport von TDA in geschmolzenem Zustand gegeben, wo inerte Gase zur Überschleierung eingesetzt werden können.

Bei dem erfindungsgemäßen Verfahren kann sowohl die Verdampfung des Amins als auch eine gegebenenfalls erforderliche Überhitzung einstufig erfolgen; sie können aber auch jeweils mehrstufig erfolgen. Nach dem ersten bzw. gegebenenfalls einzigen Überhitzer kann der dampfförmige Aminstrom durch einen Tropfenabscheider geführt werden. Alternativ ist es auch möglich, bereits nach dem Verdampfer den dampfförmigen Aminstrom durch einen Tropfenabscheider zu führen.

Die mittlere Verweilzeit vom Verlassen des Verdampfungsraumes bis zum Eintritt in den Reaktionsraum beträgt mehr als 0,01 Sekunden, bevorzugt mehr als 0,1 Sekunden und insbesondere bevorzugt mehr als 0,5 Sekunden. Bevorzugt beträgt die Verweilzeit bis zum Eintritt in den Reaktionsraum nicht mehr als 60 Sekunden. Diese Verweilzeit ist bei den in der Verdampfung gegebenen Temperaturen ausreichend lang, um zu gewährleisten, dass der in dem zur Verdampfung zugeführten inerten Verdünnungsmittel vorhandene Sauerstoff vollständig mit dem Amin reagiert.

Die Verdampfer und / oder Überhitzer sowie die Rohrleitungen zur Erzeugung des dampfförmigen Diaminstroms zum Gasphasenreaktor können aus einem beliebigen metallischen Werkstoff gefertigt sein, z. B. Stahl, Edelstahl, Titan, Hastelloy, Inconel oder anderen metallischen Legierungen.

Die Umsetzung des primären Amines mit Phosgen erfolgt in mindestens einem, in einem Reaktor angeordneten Reaktionsraum. Unter Reaktionsraum wird der Raum verstanden, in dem die Gasphasenreaktion von primärem Amin (bzw. Zwischenprodukten) mit Phosgen zum gewünschten Isocyanat stattfindet.. Unter Reaktor wird die technische Vorrichtung verstanden, die den Reaktionsraum enthält. Ein Reaktor kann dabei auch mehrere Reaktionsräume enthalten. Auch können mehrere Reaktoren mit jeweils einem oder mehreren Reaktionsräumen hintereinander oder parallel geschaltet werden.

Das erfindungsgemäße Verfahren ist prinzipiell auf jede Reaktionsraum- und Reaktorgeometrie anwendbar.

In einer weiteren bevorzugten Ausführungsform des erfindungsgemäßen Verfahrens weist der Reaktor im Anschluss an den Reaktionsraum, in dem nach der Mischung der Edukte ein Umsatz der Amingruppen zu den Isocyanatgruppen von 80,0 %, bevorzugt 90,0 %, besonders bevorzugt 99,0 %, ganz besonders bevorzugt 99,5 % erzielt wird, einen weiteren, rotationssymmetrischen Reaktionsraum mit konstanter und / oder erweiterter durchströmter Querschnittsfläche auf.

Das erfindungsgemäße Verfahren ist prinzipiell auf jede Fahrweise der Gasphasenphosgenierung anwendbar. Bevorzugt ist die in EP 1 935 876 A1 beschriebene adiabate Fahrweise. Das beschriebene Verfahren ist jedoch auch in einer isothermen Fahrweise anwendbar.

Die gewählte Verweilzeit für die Reaktion der Amingruppen mit dem Phosgen zu dem Isocyanat liegt bevorzugt zwischen 0,050 Sekunden und 15 Sekunden, in Abhängigkeit von der Art des eingesetzten Amins, der Starttemperatur, ggf. der adiabaten Temperaturerhöhung im Reaktionsraum, dem molaren Verhältnis von eingesetztem Amin und Phosgen, Art und Menge des mindestens einen inerten Stoffes sowie dem gewählten Reaktionsdruck.

Beim erfindungsgemäßen Verfahren ist es vorteilhaft, Phosgen im Überschuss bezüglich der umzusetzenden Amingruppen einzusetzen. Bevorzugt liegt ein molares Verhältnis von Phosgen zu Amingruppen von 1,1 : 1 bis 20 : 1, besonders bevorzugt von 1,2 : 1 bis 5 : 1, vor. Das Phosgen wird auf Temperaturen von 200 °C bis 600 °C erhitzt und gegebenenfalls verdünnt mit einem Inertgas wie N₂, He, Ar oder mit den Dämpfen eines inerten Lösungsmittels, z. B. aromatischen Kohlenwasserstoffen ohne oder mit Halogensubstitution, wie z. B. Chlorbenzol oder Dichlorbenzol, dem Reaktionsraum zugeführt. Wenn das Phosgen ebenfalls mit einem Verdünnungsmittel versetzt wird, so muss dessen Sauerstoffgehalt möglichst gering gehalten werden (in der Regel geringer als der Sauerstoffgehalt des Verdünnungsmittels des Amins), denn Sauerstoff im Phosgen kann zur Bildung von Rückständen im Reaktionsraum oder sogar zur Bildung von explosionsfähigen Gemischen nach der sog. Quenche (siehe unten) führen. Bevorzugt wird daher rezyklierter und damit sauerstofffreier inerter Stoff für die Verdünnung des Phosgens eingesetzt.

Nach der erfolgten Phosgenierungsreaktion im Reaktionsraum wird bevorzugt das gasförmige Reaktionsgemisch, das bevorzugt wenigstens ein Isocyanat, Phosgen, einen inerten Stoff sowie Chlorwasserstoff umfasst, von dem gebildeten Isocyanat befreit. Dies kann beispielsweise so erfolgen, dass das den Reaktionsraum kontinuierlich verlassende Reaktionsgemisch, einer Kondensation in einem inerten Lösungsmittel unterzogen wird, wie sie bereits für andere Gasphasenphosgenierungen empfohlen wurde (EP 0 749 958 A1).

Bevorzugt erfolgt die Kondensation jedoch dadurch, dass der in dem erfindungsgemäßen Verfahren eingesetzte Reaktionsraum zumindest eine Zone aufweist, in die zum Abbruch der Umsetzung der eingesetzten Amine und des Phosgens zu den entsprechenden Isocyanaten ein oder mehrere geeignete Flüssigkeitsströme ("Quench-Flüssigkeiten") eingesprüht werden. Dadurch kann, wie in EP 1 403 248 A1 beschrieben, eine rasche Abkühlung der Gasgemische ohne den Einsatz kalter Flächen durchgeführt werden.

In einer besonders bevorzugten Form des erfindungsgemäßen Verfahrens ist die zumindest eine Zone (Kühlzone) in eine Quenchstufe, wie sie z. B. in EP 1 403 248 A1 offenbart wurde, integriert. In einer besonders bevorzugten Form kommen mehrere Kühlzonen zum Einsatz. Integration und Betrieb dieser zumindest zwei Kühlzonen erfolgen bevorzugt mit einer Quenchstufe. Dies ist hinsichtlich Aufbau und Betrieb in EP 1 935 875 A1 offenbart.

Statt des integrierten Verbundes von der zumindest einen Kühlzone eines Reaktors mit einer Quenchstufe, wie sie in der EP 1 935 875 A1 offenbart wurde, ist ebenfalls der entsprechende integrierte Verbund der Kühlzonen mehrerer Reaktoren mit einer Quenchstufe möglich. Bevorzugt ist jedoch der integrierte Verbund eines Reaktors mit zumindest einer Kühlzone mit einer Quenchstufe.

Die die Kondensations- bzw. Quenchstufe verlassende Lösungen bzw. Gemische werden anschließend bevorzugt destillativ aufgearbeitet und so das Isocyanat in der geforderten Reinheit gewonnen.

Das die Kondensations- bzw. Quenchstufe verlassende Gasgemisch wird bevorzugt in einer nachgeschalteten Gaswäsche mit einer geeigneten Waschflüssigkeit von restlichem Isocyanat befreit, bevorzugt anschließend in an sich bekannter Weise von überschüssigem Phosgen befreit. Dies kann mittels einer Kühlfalle, Absorption in einem inerten Lösungsmittel (z. B. Chlorbenzol oder Dichlorbenzol) oder durch Adsorption und Hydrolyse an Aktivkohle erfolgen. Das die Phosgenrückgewinnungsstufe durchlaufende Chlorwasserstoffgas kann in an sich bekannter Weise zur Rückgewinnung des zur Phosgensynthese erforderlichen Chlors rezyklisiert werden. Die nach ihrem Einsatz zur Gaswäsche anfallende Waschflüssigkeit kann dann bevorzugt zumindest teilweise als Quench-Flüssigkeit zur Abkühlung des Gasgemisches in die entsprechende Zone des Reaktionraumes eingesetzt werden.

Sofern als Verdünnungsmittel ein inertes Gas und nicht der Dampf eines inerten Lösungsmittels eingesetzt wurde, durchläuft das inerte Gas die Kondensations- bzw. Quenchestufen sowie die gegebenenfalls nachgeschaltete Gaswäsche und Phosgenrückgewinnung gasförmig zusammen mit dem gasförmigen Chlorwasserstoff. Der gasförmige Chlorwasserstoff kann z. B. durch Absorption in Wasser abgetrennt, und so das in der Verdampfung eingesetzte inerte Gas wiedergewonnen werden. In einer bevorzugten Ausführungsform des erfindungsgemäßen Verfahrens kann dieser Strom des inerten Gases - gegebenenfalls nach Trocknung sowie gegebenenfalls nach Zerstörung von verbliebenen Spuren von Phosgen - zur Erleichterung der Überführung des Amins in die Gasphase in den Verdampfungsraum zurückgeführt werden. Dieser Strom des inerten Gases ist dabei frei von Sauerstoff, da ursprünglich vorhandener Sauerstoff bereits mit dem Amin vor dem Reaktor zur Gasphasenphosgenierung reagiert hat. Eventuelle Verluste an inertem Gas können mit frischem Verdünnungsmittel, welches vorzugsweise das gleiche inerte Gas enthält, ergänzt werden, wobei das zur Ergänzung eingesetzte frische Verdünnungsmittel einen hohen Sauerstoffgehalt von bis zu 10 Massen-% aufweisen kann, da dieser Gasstrom mit dem sauerstofffreien rezyklisierten Gas verdünnt wird. In jedem Falle muss der Gehalt an Sauerstoff in dem als Verdünnungsmittel eingesetzten inertem Gas so beschaffen sein, dass das erfindungsgemäße molare Verhältnis von Amin zu Sauerstoff von ≥ 1.000 : 1, bevorzugt im Bereich zwischen 1.000 : 1 und 1.000.000 : 1, besonders bevorzugt zwischen 10.000 : 1 und 50.000 : 1, eingehalten wird. Bei dieser Ausführungsform enthält demnach das in Schritt (i) (Überführung des Amins in die Gasphase) eingesetzte Verdünnungsmittel zumindest teilweise den nach der in Schritt (ii) erfolgten Umsetzung zum Isocyanat rezyklisierten inerten Stoff.

In dem erfindungsgemäßen Verfahren werden bevorzugt solche primären Amine eingesetzt, die im Wesentlichen ohne Zersetzung in die Gasphase überführt werden können.

Beispiele für bevorzugte aliphatische bzw. cycloalipahtische Amine sind 1,4-Diaminobutan, 1,6-Diaminohexan (HDA), 1,11-Diaminoundecan, 1-Amino-3,5,5-trimethyl-3-aminomethylcyclohexan (IPDA), 4,4'-Diaminodicyclohexylmethan oder 4,4'-Diaminodicyclohexylpropan-2,2. Besonders bevorzugt sind jedoch Diamine der vorstehend genannten Art mit ausschließlich aliphatisch oder cycloaliphatisch gebundenen Aminogruppen wie Isophorondiamin (IPDA), Hexamethylendiamin (HDA) oder Bis(p-aminocyclohexyl)methan (PACM 20).

Beispiele für bevorzugte aromatische Amine sind Toluylendiamin (TDA), insbesondere 2,4-TDA und 2,6-TDA und Gemische daraus, Diaminobenzol, Naphthylendiamin (NDA) und 2,2'-, 2,4'- oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus. Ganz besonders bevorzugt sind 2,4- / 2,6-TDA-Isomerengemische der Isomerenverhältnisse 80/20 und 65/35.

### Beispiele

### Allgemeine Bedingungen in allen Beispielen

Es stehen getrocknete Luft, sowie vier Stickstoffquellen unterschiedlicher Reinheit als Verdünnungsmittel zur Verfügung:

| | |
|---|---|
| Beispiel 1: | > 99,999%ige Reinheit (< 0,0001 Massen-% O₂) |
| Beispiel 2: | 99,32%ige Reinheit (0,68 Massen-% O₂) |
| Beispiele 3 und 4: | 98,09%ige Reinheit (1,91 Massen-% O₂) |
| Beispiel 5: | 92,12%ige Reinheit (7,88 Massen-% O₂) |
| Beispiel 6: | getrocknete Luft (78 Massen-% N₂, 22 Massen-% O₂) |

Unter Einsatz dieser Verdünnungsmittel soll ein m-Toluylendiamin-Isomerengemisch enthaltend 80,3 % 2,4-TDA und 19,7 % 2,6-TDA verdampft werden. Dazu werden ca. 100 g des TDA-Isomerengemisches genau in einen 250-mL-Rundkolben eingewogen, der an eine im Unterdruck betreibbare Destillationsapparatur, bestehend aus Reitmeyer-Aufsatz, Brücke und gekühlter Destillationsvorlage, angeschlossen ist. In den Rundkolben ragt eine Siedekapillare, die unmittelbar oberhalb des Flüssigkeitsstandes des eingefüllten TDA-Isomerengemisches endet. Über diese Siedekapillare wird das Verdünnungsmittel mit 4 bzw. 10 L/h (bei 1013hPa und 20°C) in den Rundkolben eingeleitet, während dieser mittels eines Heißluftgebläses erhitzt wird. Der sich bildende TDA-Dampf wird zusammen mit dem Verdünnungsmittel bei einem Absolutdruck von 100 mbar durch Reitmeyeraufsatz und Brücke in die Destillationsvorlage geführt, dort kondensiert und aufgefangen. Die Destillation wird solange betrieben, bis das vorgelegte TDA vollständig verdampft ist. Dies ist bei dem eingestellten absoluten Druck von 100 mbar nach einer Zeit von 16 bis 17 Minuten der Fall und auch daran zu erkennen, dass die Innentemperatur im Rundkolben 180 °C übersteigt. Durch die Dauer der Destillation und den bekannten Volumenstrom des Verdünnungsmittels kann die molare Menge an Sauerstoff berechnet werden, mit dem der TDA-Dampf in Kontakt kam.

Bilden sich durch den Kontakt mit TDA-Dampf und Sauerstoff Oxidationsprodukte, deren Siedepunkt nicht wesentlich höher ist als der von m-TDA, so gelangen diese bis in die Destillationsvorlage, wo ihr Gehalt durch eine Rückstandsdestillation des aufgefangenen Destillats bestimmt werden kann.

Bilden sich durch den Kontakt mit TDA-Dampf und Sauerstoff Oxidationsprodukte, deren Siedepunkt wesentlich höher ist als der von m-TDA, so verbleiben diese im Rundkolben bzw. im Reitmeyer-Aufsatz und können dort zurückgewogen werden.

### Beispiel 1 (Vergleichsbeispiel: Sauerstoffgehalt des Verdünnungsmittels < 0,0001 Massen-%; molares Verhältnis Amin : Sauerstoff > 1000 : 1)

Während der 17minütigen Destillation von 101,6 g m-TDA (0,83 mol) wurden 10 L/h Stickstoff als Verdünnungsmittel zu dem TDA-Dampf gegeben. Der Stickstoff wies entsprechend dem Stand der Technik zur Gasphasenphosgenierung einen Sauerstoffgehalt von < 0,0001 Massen-% (entspricht 1 ppm), nämlich von 0,62 ppm auf. Die eingebrachte Sauerstoffmenge ist somit vernachlässigbar klein, so dass das molare Verhältnis von m-TDA zu Sauerstoff wesentlich größer als 1.000 : 1 ist.

Die Mengen an Rückstand im Rundkolben betrug 1300 mg, die Menge an Rückstand in der Destillationsvorlage 70 mg.

### Beispiel 2 (Erfindungsgemäß: Sauerstoffgehalt des Verdünnungsmittels > 0,0001 Massen-%; molares Verhältnis Amin : Sauerstoff > 1000 : 1)

Es wird ebenso wie in Beispiel 1 vorgegangen. Während der 16,25minütigen Destillation von 109,4 g m-TDA (0,90 mol) wurden 10 L/h Stickstoff mit **0,68 Massen-% Sauerstoff** eingesetzt. Das molare Verhältnis von Amin zu Sauerstoff beträgt rechnerisch 1173 : 1. Die Mengen an Rückstand im Rundkolben betrug 1286 mg, die Menge an Rückstand in der Destillationsvorlage 73 mg. Die Rückstandswerte sind praktisch identisch zu denen in Beispiel 1, die Ausbeute ist also ebenso gut wie beim Einsatz des reinen Stickstoffs.

### Beispiel 3 (erfindungsgemäß: Sauerstoffgehalt des Verdünnungsnüttels > 0,0001 Massen-%; molares Verhältnis Amin : Sauerstoff > 1000 : 1 - Reduzierung der Absolutmenge an Verdünnungsmittel)

Es wird ebenso wie in Beispiel 1 vorgegangen. Während der 16,25minütigen Destillation von 106,2 g m-TDA (0,87 mol) wurden lediglich 4 L/h Stickstoff mit **1,91 Massen-% Sauerstoff** eingesetzt. Durch die kleinere Menge an Verdünnungsmittel ist das molare Verhältnis von Amin zu Sauerstoff nach wie vor größer als 1000, nämlich rechnerisch 1010 : 1. Die Mengen an Rückstand im Rundkolben betrug 1296 mg, die Menge an Rückstand in der Destillationsvorlage 72 mg. Beide Rückstandswerte sind ebenso niedrig wie bei Verwendung des reinen Sauerstoffs in Beispiel 1.

### Beispiel 4 (Vergleichsbeispiel: Sauerstoffgehalt des Verdünnungsmittels > 0,0001 Massen-%; molares Verhältnis Amin : Sauerstoff < 1000 : 1)

Es wird ebenso wie in Beispiel 3 vorgegangen. Während der 17minütigen Destillation von 105,6 g m-TDA (0,86 mol) wurden 10 L/h Stickstoff mit **1,91 Massen-% Sauerstoff** eingesetzt. Das molare Verhältnis von Amin zu Sauerstoff beträgt jetzt rechnerisch 384 : 1. Die Mengen an Rückstand im Rundkolben betrug 1408 mg, die Menge an Rückstand in der Destillationsvorlage 81 mg. Beide Rückstandswerte sind gegenüber Beispiel 1 bis 3 signifikant erhöht, was sich bei der großtechnischen Durchführung als Ausbeutverlust bemerkbar machen würde. Das Beispiel zeigt, dass sich die Veränderung des molaren Verhältnisses von m-TDA zu Sauerstoff auf einen Wert von kleiner 1000 negativ auf die Ausbeute auswirkt.

### Beispiel 5 (Vergleichsbeispiel: Sauerstoffgehalt des Verdünnungsmittels > 0,0001 Massen-%; molares Verhältnis Amin : Sauerstoff < 1000 : 1)

Es wird ebenso wie in Beispiel 1 vorgegangen. Während der 17minütigen Destillation von 106,0 g m-TDA (0,87 mol) wurden 10 L/h Stickstoff mit **7,88 Massen-% Sauerstoff** eingesetzt. Das molare Verhältnis von Amin zu Sauerstoff beträgt rechnerisch 93 : 1. Die Mengen an Rückstand im Rundkolben betrug 1393 mg, die Menge an Rückstand in der Destillationsvorlage 91 mg. Beide Rückstandswerte sind gegenüber Beispiel 1 bis 3 signifikant erhöht, was sich bei der großtechnischen Durchführung als Ausbeuteverlust bemerkbar machen würde. Gegenüber Beispiel 1 ist der Rückstand in der Destillationsvorlage (Gehalt an Oxidationsprodukten, deren Siedepunkt nicht wesentlich höher ist als der von m-TDA) um 30 % erhöht, was bei der großtechnischen Durchführung dieses Verfahrens neben Ausbeuteverluste zu beschleunigter Verstopfung der TDA-Dampf führenden Leitungen führen kann.

### Beispiel 6 (Vergleichsbeispiel: Sauerstoffgehalt des Verdünnungsmittels 22 Massen-%; molares Verhältnis Amin : Sauerstoff < 1000 : 1)

Während der 17minütigen Destillation von 112,5 g m-TDA (0,92 mol) wurden 10 L/h getrocknete Luft als Verdünnungsmittel zu dem TDA-Dampf gegeben. Das molare Verhältnis von Amin zu Sauerstoff beträgt rechnerisch 37 : 1. Es kam zur Bildung von Oxidationsprodukten deren Siedepunkt nicht wesentlich höher ist als der von m-TDA, was in einer deutlichen Verfärbung des in der Destillationsvorlage aufgefangenen m-TDA zu beobachten war. Auch kam es zur Bildung von Oxidationsprodukten mit wesentlich höherem Siedepunkt, die als Rückstand in dem Rundkolben zurückbleiben.

Die Mengen an Rückstand im Rundkolben betrug 1477 mg, die Menge an Rückstand in der Destillationsvorlage 113 mg. Dies bedeutet im Vergleich zu Beispiel 1 einen Anstieg von 13 % im Rundkolben (Oxidationsprodukte mit wesentlich höherem Siedepunkt als m-TDA) und von 61 % in der Destillationsvorlage (Oxidationsprodukte, deren Siedepunkt nicht wesentlich höher ist als der von m-TDA). Der deutliche Anstieg an Oxidationsprodukten, deren Siedepunkt nicht wesentlich höher ist als der von m-TDA, würde bei der großtechnischen Durchführung dieses Verfahrens neben Ausbeuteverluste zu beschleunigter Verstopfung der TDA-Dampf führenden Leitungen führen.

Tabelle 1 fasst für die Beispiele 1 bis 6 zusammen, wie die Bildung von Oxidationsprodukten mit ähnlichem bzw. wesentlich höherem Siedepunkt als m-TDA von dem Verhältnis von m-TDA zu Sauerstoff während der Destillation abhängt.

**Tabelle 1:**

| Beispiel | molares Verhältnis m-TDA : Sauerstoff | Menge Verdünnungsmittel [L/h] | Sauerstoffgehalt Verdünnungsmittel [Massen-%] | Rückstand im Destillationssumpf [mg] | Rückstand im Destillat [mg] |
|---|---|---|---|---|---|
| 1 | >> 1000 | 10 | < 0,0001 | 1302 | 70 |
| 2 | 1173 | 10 | 0,68 | 1286 | 73 |
| 3 | 1010 | 4 | 1,91 | 1296 | 72 |
| 4 | 384 | 10 | 1,91 | 1408 | 81 |
| 5 | 93 | 10 | 7,88 | 1393 | 91 |
| 6 | 37 | 10 | 22 | 1477 | 113 |

## Patentansprüche

1. Verfahren zur Herstellung von Isocyanaten durch Umsetzung der korrespondierenden Amine mit Phosgen in der Gasphase, worin
(i) das Amin in einem Verdampfungsraum in Gegenwart von > 0,10 Massen-% bis 25 Massen-% eines Verdünnungsmittels, bezogen auf die Gesamtmasse aus Verdünnungsmittel und Amin, in die Gasphase überführt wird;
(ii) der aus (i) erhaltene, Amin und Verdünnungsmittel enthaltende gasförmige Strom mit einem gasförmigen Phosgenstrom in einem Reaktionsraum zum entsprechenden Isocyanat umgesetzt wird;
**dadurch gekennzeichnet, dass**
das in Schritt (i) eingesetzte Verdünnungsmittel zwischen 90,0000 Massen-% und 99,9999 Massen-% an im Phosgenierprozess inerten Stoffen und zwischen 0,0001 Massen-% und 10,0000 Massen-% Sauerstoff, jeweils bezogen auf die Masse des Verdünnungsmittels, enthält, und
das molare Verhältnis von Amin zu Sauerstoff (O₂) in Schritt (i) im Verdampfungsraum ≥ 1.000 : 1 ist.

2. Verfahren nach Anspruch 1, bei dem in Schritt (i) das Amin in Gegenwart von > 0,10 Massen-% bis 10 Massen-% eines Verdünnungsmittels, welches zwischen 99,0000 Massen-% und 99,9950 Massen-% an im Phosgenierprozess inerten Stoffen und zwischen 0,0050 Massen-% und 1,0000 Massen-% Sauerstoff, jeweils bezogen auf die Masse des Verdünnungsmittels, enthält, in die Gasphase überführt wird, und wobei das molare Verhältnis von Amin zu Sauerstoff im Verdampfungsraum im Bereich zwischen 1.000 : 1 und 1.000.000 : 1 liegt.

3. Verfahren nach Anspruch 1, bei dem in Schritt (i) das Amin in Gegenwart von > 0,20 Massen-% bis 5,0 Massen-% eines Verdünnungsmittels, welches zwischen 99,5000 Massen-% und 99,9900 Massen-% an im Phosgenierprozess inerten Stoffen und zwischen 0,0100 Massen-% und 0,5000 Massen-% Sauerstoff, jeweils bezogen auf die Masse des Verdünnungsmittels, enthält, in die Gasphase überführt wird, und wobei das molare Verhältnis von Amin zu Sauerstoff im Verdampfungsraum im Bereich zwischen 10.000 : 1 und 50.000 : 1 liegt.

4. Verfahren nach Anspruch 1, bei dem die Einhaltung des molaren Verhältnisses von Amin zu Sauerstoff (O₂) in Schritt (i) im Verdampfungsraum von ≥ 1.000 : 1 durch Verringerung des absoluten Mengenstromes des dem Verdampfungsraum zugeführten Verdünnungsmittels erzielt wird.

5. Verfahren nach einem der Ansprüche 1 bis 4, bei dem das in Schritt (i) eingesetzte Verdünnungsmittel zumindest teilweise nach der in Schritt (ii) erfolgten Umsetzung zum Isocyanat rezyklisierten inerten Stoff enthält.

6. Verfahren nach einem der Ansprüche 1 bis 5, bei dem das Amin Toluylendiamin (TDA) ist.

7. Verfahren nach einem der Ansprüche 1 bis 6, bei dem der im Phosgenierprozess inerte Stoff Stickstoff ist.

## Claims

1. Process for the preparation of isocyanates by reaction of the corresponding amines with phosgene in the gas phase, wherein
(i) the amine is converted into the gas phase in a vaporization space in the presence of from > 0.10 % by weight to 25 % by weight of a diluent, based on the total weight of diluent and amine;
(ii) the gaseous stream obtained from (i) containing amine and diluent is reacted with a gaseous phosgene stream in a reaction space to give the corresponding isocyanate;
**characterized in that**
the diluent employed in step (i) contains between 90.0000 % by weight and 99.9999 % by weight of substances which are inert in the phosgenation process and between 0.0001 % by weight and 10.0000 % by weight of oxygen, in each case based on the weight of the diluent,
and
the molar ratio of amine to oxygen (O₂) in step (i) in the vaporization space is ≥ 1,000 : 1.

2. Process according to Claim 1, in which in step (i) the amine is converted into the gas phase in the presence of > 0.10 % by weight to 10 % by weight of a diluent which contains between 99.0000 % by weight and 99.9950 % by weight of substances which are inert in the phosgenation process and between 0.0050 % by weight and 1.0000 % by weight of oxygen, in each case based on the weight of the diluent, and wherein the molar ratio of amine to oxygen in the vaporization space is in the range between 1,000 : 1 and 1,000,000 : 1.

3. Process according to Claim 1, in which in step (i) the amine is converted into the gas phase in the presence of > 0.20 % by weight to 5.0 % by weight of a diluent which contains between 99.5000 % by weight and 99.9900 % by weight of substances which are inert in the phosgenation process and between 0.0100 % by weight and 0.5000 % by weight of oxygen, in each case based on the weight of the diluent, and wherein the molar ratio of amine to oxygen in the vaporization space is in the range between 10,000 : 1 and 50,000 : 1.

4. Process according to Claim 1, in which the molar ratio of amine to oxygen (O₂) in step (i) in the vaporization space of ≥ 1,000 : 1 is maintained by reducing the absolute volume flow of the diluent fed to the vaporization space.

5. Process according to one of Claims 1 to 4, in which the diluent employed in step (i) at least partially contains inert substance recycled after the reaction to give the isocyanate has taken place in step (ii).

6. Process according to one of Claims 1 to 5, in which the amine is toluylenediamine (TDA).

7. Process according to one of Claims 1 to 6, in which the substance which is inert in the phosgenation process is nitrogen.

## Revendications

1. Procédé de fabrication d'isocyanates par mise en réaction des amines correspondantes avec du phosgène en phase gazeuse, selon lequel
(i) l'amine est transférée dans la phase gazeuse dans une chambre de vaporisation en présence de > 0,10 % en masse à 25 % en masse d'un diluant, par rapport à la masse totale de diluant et d'amine ;
(ii) le courant gazeux contenant l'amine et le diluant obtenu en (i) est mis en réaction avec un courant de phosgène gazeux dans une chambre de réaction pour former l'isocyanate correspondant ;
**caractérisé en ce que**
le diluant utilisé à l'étape (i) contient entre 90,0000 % en masse et 99,9999 % en masse de matières inertes dans le procédé de phosgénation et entre 0,0001 % en masse et 10,0000 % en masse d'oxygène, à chaque fois par rapport à la masse du diluant, et
le rapport molaire entre l'amine et l'oxygène (O₂) à l'étape (i) dans la chambre de vaporisation est de ≥ 1 000:1.

2. Procédé selon la revendication 1, selon lequel, à l'étape (i), l'amine est transférée dans la phase gazeuse en présence de > 0,10 % en masse à 10 % en masse d'un diluant, qui contient entre 99,0000 % en masse et 99,9950 % en masse de matières inertes dans le procédé de phosgénation, et entre 0,0050 % en masse et 1,0000 % en masse d'oxygène, à chaque fois par rapport à la masse du diluant, et le rapport molaire entre l'amine et l'oxygène dans la chambre de vaporisation se situe dans la plage comprise entre 1 000:1 et 1 000 000:1.

3. Procédé selon la revendication 1, selon lequel, à l'étape (i), l'amine est transférée dans la phase gazeuse en présence de > 0,20 % en masse à 5,0 % en masse d'un diluant, qui contient entre 99,5000 % en masse et 99,9900 % en masse de matières inertes dans le procédé de phosgénation, et entre 0,0100 % en masse et 0,5000 % en masse d'oxygène, à chaque fois par rapport à la masse du diluant, et le rapport molaire entre l'amine et l'oxygène dans la chambre de vaporisation se situe dans la plage comprise entre 10 000:1 et 50 000:1.

4. Procédé selon la revendication 1, selon lequel le maintien du rapport molaire entre l'amine et l'oxygène (O₂) à l'étape (i) dans la chambre de vaporisation de ≥ 1 000:1 est obtenu par réduction du débit massique absolu du diluant introduit dans la chambre de vaporisation.

5. Procédé selon l'une quelconque des revendications 1 à 4, selon lequel le diluant utilisé à l'étape (i) contient une matière inerte recyclée au moins en partie après la transformation en isocyanate qui a lieu à l'étape (ii).

6. Procédé selon l'une quelconque des revendications 1 à 5, selon lequel l'amine est la toluylène-diamine (TDA).

7. Procédé selon l'une quelconque des revendications 1 à 6, selon lequel la matière inerte dans le procédé de phosgénation est l'azote.
